# EUROPEAN PATENT APPLICATION

(11) **EP 4 613 266 A1**
(43) Date of publication of application: **10.09.2025**
(21) Application number: 23884874.1
(22) Date of filing: 30.10.2023
(51) Int. Cl.: A61K 9/14, A61K 31/12, A61K 47/10, A61K 47/38, A61K 47/32, A61K 9/48, A61K 9/20, A61P 9/00, A61P 13/12

(54) **SOLID DISPERSION OF CURCUMIN DERIVATIVE, METHOD FOR PREPARING SAME, AND USE THEREOF**

(30) Priority: 04.11.2022 CN 202211375466
(71) Applicant: Wenzhou Medical University, Wenzhou, Zhejiang 325035 (CN)
(72) Inventor: LIANG, Guang, Wenzhou, Zhejiang 325035 (CN); WU, Wenqi, Wenzhou, Zhejiang 325035 (CN); WANG, Yi, Wenzhou, Zhejiang 325035 (CN)
(74) Representative: karo IP
(86) International application number: PCT/CN2023/127924
(87) International publication number: WO 2024/093944

(57) **Abstract**

The present invention relates to the field of pharmaceutical chemicals, and in particular, to a solid dispersion of a curcumin derivative C66, a method for preparing same, and use thereof. In the solid dispersion described by the present invention, C66 is dispersed in a carrier material in a microcrystalline or amorphous form. Compared with the C66 substance, the C66 solid dispersion features significantly improved dissolution rate of C66 in a dissolution medium. In a Beagle dog oral bioavailability study, the absolute bioavailability of the C66 solid dispersion was 6.66 times that of the C66 substance. Meanwhile, the C66 solid dispersion is good in stability, and after a 6-month acceleration study, the dissolution rate, related substances, isomers, and the like met the quality control requirements. The powder features good fluidity and good formulation processing performance. Capsules can be conveniently prepared by means of a direct powder filling process, and tablets can be prepared by means of a direct powder pressing process. This lays a foundation for further development of C66 formulation products.

## Description

### TECHNICAL FIELD

The present invention relates to the field of pharmaceutical chemicals, and in particular, to a solid dispersion of a curcumin derivative C66, a method for preparing same, and use thereof.

### BACKGROUND

With the advent of an aging society, the incidence of cardiovascular and cerebrovascular diseases caused by chronic inflammation has been increasing. Complications caused by diabetes, obesity, hypertension, and the like, have seriously affected human health and quality of life. In case of hyperglycemia or hypertension, various tissues and organs of a body will suffer from a series of pathological changes and injuries, resulting in a variety of complications, especially kidney damage, which initially manifests as microalbuminuria. As a course of disease prolongs and urinary protein increases, the kidney's ability to eliminate toxins from the blood gradually declines, eventually developing into an end-stage renal disease, which can only be managed through hemodialysis or kidney transplantation to sustain life. With the accelerated economic development and the aging population in China, the prevalence and mortality of metabolic diseases, such as diabetes, hypertension and complications, are expected to continue rising in the future, and will become one of the most prominent public health issues in China.

At present, treatment for kidney diseases caused by diabetes and hypertension remain unsatisfactory. In an early stage of diabetic nephropathy, treatment methods still mainly focus on lowering blood glucose, blood pressure, and blood lipids to treat the diabetes. In a late stage of diabetic nephropathy, treatment of kidney diseases mainly relies on dialysis and kidney transplantation. The treatment of kidney failure in the late stage remains limited and largely palliative. In the early treatment of diabetic nephropathy, existing treatment means can slow down the kidney damage caused by diabetes but are unable to reverse the kidney damage that has already occurred, or prevent the progression of nephropathy to end-stage kidney failure. Through extensive research, the inventor has independently designed and synthesized hundreds of curcumin structural analogs using curcumin, an active ingredient of traditional Chinese medicine, as a lead compound, and a novel curcumin analog, C66, has been identified and demonstrated high efficacy in alleviating nephropathy caused by diabetes, hypertension, hyperlipidemia, and obesity. The inventor has already conducted extensive pharmacological mechanism studies and druggability evaluation of C66 in the early stage, and has found that C66 exhibited high druggability. A chemical structure of C66 is shown as follows:

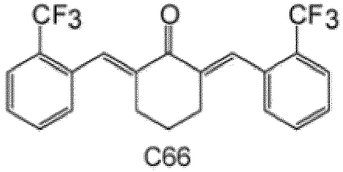

The inventor was previously granted a patent for the use of C66 as a therapeutic drug for nephropathy and heart disease caused by diabetes (ZL 201110146331.2), and has also applied for patents for the use of C66 as a therapeutic drug for kidney diseases caused by obesity, hypertension, and hyperlipidemia (CN 201910619696.9) and for a compound crystalline form (CN 201910831988.9), and the like.

### SUMMARY

### A technical problem to be solved by the present invention is as follows:

In previous studies, the inventor found that C66 had good permeability, but its water solubility was poor. Experimental data of the studies are shown in Table 1 below:

**Table 1. Solubility of C66**

| S/N | Solvent (Medium) | Result |
|---|---|---|
| 1 | Purified water | Almost insoluble or insoluble |
| 2 | Ethanol (95%) | Slightly soluble |
| 3 | Hydrochloric acid solution with a pH of 1.0 | Almost insoluble or insoluble |
| 4 | Phosphate buffer with a pH of 6.8 | Almost insoluble or insoluble |

The inventor also investigated the solubility of C66 in sodium dodecyl sulfate (SDS) and Tween 80 (T80). According to the recommended usage of surfactants specified in *Handbook of Pharmaceutical Excipients,* and recommendations from the National Medical Products Administration of China and the U.S. Food and Drug Administration (FDA), a proportion of surfactants in the solution is 1%-3%. A saturated concentration of C66 in a surfactant solutions is shown in Table 2:

**Table 2 Solubilization effect of SDS and Tween 80 on C66**

| Name | Saturated concentration of C66 (µg/mL) |
|---|---|
| 1% SDS solution | 5.158 |
| 2% SDS solution | 9.046 |
| 3% SDS solution | 22.484 |
| 1% Tween 80 solution | 1.410 |
| 2% Tween 80 solution | 3.385 |
| 3% Tween 80 solution | 15.231 |

From the above results, it can be seen that the solubility of C66 substance in water is poor, but surfactants have an improving effect. Both SDS and Tween can increase the solubility of C66 in water, with SDS showing better solubilization effect than Tween 80 at the same concentration. However, in general, a 3% SDS solution shows a maximum saturated concentration of C66, which is only 22.484 µg/mL, that is, only 22.484 mg is dissolved in 1000 mL of dissolution medium. Therefore, even when a 10 mg prescription capsule is made, it is unable to reach a sink condition of 3-10 times.

In addition, the inventor also prepared a series of buffer solutions (pH 1.0, pH 1.2, pH 2.0, pH 4.0, pH 5.0, pH 6.0, pH 6.8, and pH 7.4) according to the recommended conditions of the *Pharmacopoeia,* to investigate the solubility of C66 under different pH conditions. Excess C66 powder was added to each of the solutions to obtain a mixture, the mixture was shaken in a 37°C shaker for 24 h. The C66 concentration was then measured using high-performance liquid chromatography. Results indicated that C66 was not dissolved in any of the above solutions.

To further investigation the effect of low solubility of C66 on oral absorption, the inventor conducted an oral bioavailability study. 6 male Beagle dogs (with a weight of 8.00-8.62 kg per dog) that passed the quarantine were selected and divided into 2 groups by weight according to a completely random method, that is, oral administration group, and intravenous injection group, with 3 dogs in each group, and single gavage administration were given. The dogs were subjected to fasting but not water deprivation 12 h before administration, and were provided with food 4 h after administration, with free access to water. For the oral administration group, 1% sodium carboxymethyl cellulose (CMC-Na) was mixed with C66 substance to prepare a 0.6 mg/mL suspension for gavage. For the intravenous injection group, a required amount of C66 substance was weighed and dissolved in a solution containing 5% DMSO + 10% polyethylene glycol-15 hydroxystearate + 85% (20% 2-hydroxypropyl-(3-cyclodextrin) to prepare a 1 mg/mL clear solution. Blood samples were collected at different time points after administration, and a concentration of C66 in the blood samples were detected by a liquid chromatograph mass spectrometer to obtain a drug concentration-time curve. An area under the curve (AUC) was analyzed, and analysis results indicated that an absolute bioavailability of oral C66 was only 7.16%, with an AUC of the intravenous injection group being 100%.

In view of the extremely poor water solubility and low oral bioavailability of C66, it is necessary to perform proper solubilization treatment on C66 to improve its oral bioavailability. The present invention achieves the objective by using solid dispersion technology. Since C66 is a Class 1.1 Innovative Drug that is under research and development by the inventor, neither literature nor patent reports on solid dispersions of C66 is available at present.

Therefore, a technical problem to be solved by the present invention is as follows:
the present invention provides a solid dispersion of a curcumin derivative C66, a method for preparing same, and use thereof. By preparing the solid dispersion, water solubility and oral bioavailability of C66 are effectively improved, making it more suitable for pharmaceutical formulations. In addition, the solid dispersion overcomes the shortcomings of conventional solid dispersions, such as poor physical stability and susceptibility to aging.

A technical solution adopted by the present invention as follows:
a solid dispersion of a curcumin derivative, including a curcumin derivative and a carrier material;
the curcumin derivative is C66, with a structure shown in Formula (I): 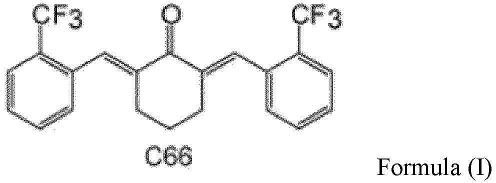
where the carrier material includes but is not limited to one, two, or three of polyethylene glycol 6000, povidone K30, copovidone S630, hydroxypropyl methylcellulose acetate succinate (HPMCAS), hydroxypropyl methylcellulose (HPMC); and the curcumin derivative is dispersed in the carrier material in a microcrystalline or amorphous form.

In the present invention, the curcumin derivative is uniformly dispersed in a specific carrier material in a microcrystalline or amorphous form, the resulting solid dispersion can effectively improve water solubility and bioavailability of the curcumin derivative, and water solubility of the curcumin derivative can be maintained for a prolonged period of time, overcoming the common shortcomings of other conventional solid dispersions susceptible to aging.

Preferably, a mass ratio of the curcumin derivative to the carrier material is 1:0.5 to 1:25. Preferably, the carrier material of the solid dispersion is polyethylene glycol 6000 and copovidone S630. A ratio of C66 to the carrier material can be 1:0.5 to 1:20.

Preferably, a ratio of C66 to polyethylene glycol 6000 is 1:9 to 1:15, and a ratio of C66 to copovidone S630 is 1:5 to 1:20.

A C66 solid dispersion prepared by using polyethylene glycol 6000 as a carrier is a microcrystalline solid dispersion, where C66 is dispersed in the polyethylene glycol 6000 in a form of micro/fine crystals; and a C66 solid dispersion prepared by using copovidone S630 or povidone K30 as a carrier is an amorphous solid dispersion, where C66 is dispersed in the carrier material in an amorphous form.

Preferably, a method for dispersion is a spray drying method, a hot-melt extrusion method, or a melt method for dispersion.

Specifically, a process of the spray drying method is as follows: completely dissolving or dispersing a curcumin derivative and a carrier material in a solvent, removing the solvent using a spray dryer to obtain a dry solid, and sieving the dry solid to obtain the solid dispersion.

A process of the hot-melt extrusion method is as follows: physically mixing a carrier material and a curcumin derivative to obtain a mixture, then extruding the mixture using a hot-melt extruder to prepare a sample, and pulverizing and sieving the sample to obtain the solid dispersion.

A process of the melt method for dispersion is as follows: heating and melting a carrier material, then adding a curcumin derivative for melting and dispersion to obtain a mixture, cooling the mixture, and pulverizing and sieving obtain the solid dispersion.

The present invention further provides a method for preparing the C66 solid dispersion, including the following steps: weighing an appropriate amount of C66 substance, povidone K30 or hydroxypropyl methylcellulose acetate succinate, placing them into a stoppered conical flask; weighing and adding 50 mL of an organic solvent to the stoppered conical flask, sealing the stoppered conical flask tightly, and performing ultrasonic shaking until the mixture in the flask becomes clear to obtain a mixed solution for later use; performing spray drying using a spray dryer, with an inlet air temperature of 60-65°C; pumping the mixed solution through a peristaltic pump for atomization and drying, with a peristaltic pump feed rate of 20 mL/min, and an outlet air temperature of 35-40°C; and collecting a sample using a cyclone collector to obtain C66-PVP K30 solid dispersion or C66-HPMCAS solid dispersion, where a C66 content in the C66-PVP K30 solid dispersion is 30-40%, and a C66 content in the C66-HPMCAS solid dispersion is 15-20%. The organic solvents include but are not limited to one or more of methanol, ethanol, acetone, isopropanol, ethyl acetate, toluene, 1,4-dioxane, acetonitrile, methyl tert-butyl ether, isopropyl acetate, heptane, and methyl isobutyl ketone.

Preferably, the organic solvent is acetone or ethanol.

Alternatively, the organic solvent is a mixed solvent of acetone and ethanol; and
a volume ratio of acetone to ethanol is 1:0.5 to 1:1.5.

The present invention also provides another method for preparing the C66 solid dispersion, including the following steps: weighing a prescribed amount of polyethylene glycol (PEG) 6000 and placing into a beaker, heating in a water bath, stirring until fully dissolved, then adding a prescribed amount of C66, stirring until it is completely melted, and keeping it warm for a period of time; spreading the sample evenly on a stainless steel tray while still hot, and cooling naturally until the sample is solidified, pulverizing and sieving to obtain the C66 solid dispersion.

Preferably, a ratio of C66 to polyethylene glycol 6000 is 1:1 to 1:15; more preferably, the ratio of C66 to polyethylene glycol 6000 is 1:12 to 1:15; and most preferably 1:12. Preferably, the water bath temperature is 70°C - 90°C.

Preferably, the heat preservation time is 0.5 - 2.0 h.

The present invention further provides a method for preparing the C66 solid dispersion on a large scale, including the following steps: weighing an appropriate amount of C66 substance and copovidone S630, placing them in a sampling bag, premixing them and sieving through a 40-mesh sieve, and continuing to mix to obtain a premixed powder; preparing a solid dispersion using a hot-melt extruder, setting nozzle temperature, screw speed, and other parameters, and performing hot-melt extrusion; discharging the samples from the nozzle in a form of a light yellow transparent colloid, and quickly cooling and solidifying into a light yellow transparent solid, collecting them in a stainless steel tray, and breaking into small segments, and taking appropriate amounts of the samples and pulverizing using a universal grinder and passing through a 100-mesh standard sieve to obtain the C66 solid dispersion.

Preferably, the nozzle temperature is 130°C - 150°C.

Preferably, the screw speed is 100 - 400 rpm.

The present invention also investigates the solubility of C66 substance and the C66 solid dispersion, and the oral bioavailability using Beagle dogs as model animals. In addition, the stability of the C66 solid dispersion is also studied. Related results indicate that the water solubility, oral bioavailability, and stability of the C66 solid dispersion all meet the expected requirements.

The present invention further provides use of the C66 solid dispersion in the treatment or prevention of diabetic nephropathy, hypertensive nephropathy, nephrotic syndrome, chronic glomerulonephritis, IgA nephropathy, lupus nephritis, hepatitis B-related nephritis, Henoch-Schonlein purpura nephritis, membranous nephropathy, and various primary and secondary chronic kidney diseases after kidney transplantation.

The present invention also provides two pharmaceutical formulations (capsules and tablets), where the pharmaceutical formulations contain the C66 solid dispersion and one or more pharmaceutically acceptable inert and non-toxic carriers or excipients.

Compared with the prior art, the present invention has the following beneficial effects:
The present invention identified that the solid dispersion technology can be used for solubilization of the curcumin derivative C66, providing suitable excipients, preparation methods, and appropriate formation forms capable of being used for C66 solid dispersion. Compared with the C66 substance, the solid dispersion can significantly improve the water solubility of C66, thus improving its oral bioavailability. Compared with other conventional solid dispersions, the solid dispersion of the present invention has good physical stability, overcoming the shortcomings of other conventional solid dispersions susceptible to aging. After a 6-month acceleration study, the dissolution rate, related substances, impurities, and other indicators meet the quality control requirements (according to the relevant technical guidance principles of the Center for Drug Evaluation). In addition, the solid dispersion exhibits excellent fluidity and good drugability.

### BRIEF DESCRIPTION OF THE DRAWINGS

FIG. 1 is an X-ray powder diffraction pattern of a curcumin derivative C66-povidone K30 amorphous solid dispersion according to the present invention.
FIG. 2 is an X-ray powder diffraction pattern of a curcumin derivative C66-polyethylene glycol 6000 microcrystalline solid dispersion according to the present invention.
FIG. 3 is an X-ray powder diffraction pattern of a curcumin derivative C66-copovidone S630 amorphous solid dispersion according to the present invention.

### DETAILED DESCRIPTION OF EMBODIMENTS

Specific embodiments of the present invention are described with reference to the following examples, which are intended to illustrate the present invention but are not intended to limit the present invention in any form.

### Example 1 Preparation of solid dispersion of curcumin derivative C66

(1) 6.01 g of C66 substance and 12.00 g of povidone K30 were weighed and placed into a stoppered conical flask, 50 mL of acetone and 50 mL of ethanol were respectively taken and added to the stoppered conical flask to obtain a mixture, the flask was sealed tightly, the mixture was dissolved by ultrasonic shaking until clear to obtain a mixed solution for later use;
(2) a Buchi B290 spray dryer, with a nozzle size of 1.5 mm, was used for drying, an inlet air temperature was 60-65°C, a peristaltic pump feed rate was set to 20 mL/min, and an outlet air temperature was 35-40°C; and the outlet air temperature was used as a main control parameter, and was maintained with the ranged by adjusting the inlet air temperature; and
(3) sample powder in a cyclone collector was scraped and taken, and the powder was a C66-povidone K30 solid dispersion, in which the C66 content was 33.33%; and the powder was weighed to calculate a yield, and then stored it in a sealed container.

### Example 2 Preparation of solid dispersion of curcumin derivative C66

### Prescription composition:

| Name of materials | Prescribed amount (g) | Remarks |
|---|---|---|
| C66 substance | 10 | Pass through a 65-mesh standard sieve after universal grinding |
| Polyethylene glycol 6000 | 120 | Mass ratio API: polyethylene glycol 6000 = 1: 12 |

### Process description:

(1) the prescribed amount of PEG 6000 was weighed and placed into a beaker, and heated in an 85°C water bath and stirred manually with a glass rod until completely it was fully melted;
(2) after the PEG 6000 was fully melted, the prescribed amount of active pharmaceutical ingredient (API) was added, and heated in an 85°C water bath and stirred manually with a glass rod until it was fully dissolved;
(3) after the sample was fully dissolved, and kept warm in an 85°C water batch for 30-40 min;
(4) after the heat preservation was completed, the sample was spread evenly on a stainless steel tray while still hot, and cooled naturally until the sample was solidified; and
(5) the solidified sample was pulverized and passed through an 80-mesh standard sieve to obtain the C66 solid dispersion.

### Example 3 Preparation of solid dispersion of curcumin derivative C66

120.0 g of API and 600.0 g of copovidone S630 was weighed and placed into a sample bag, mixed manually for about 1 min, and passed through a 40-mesh standard sieve, and then manually mixed again for about 2 min to obtain a pre-mixed powder; and samples were prepared using a hot-melt extruder (Process 11, Thermo), and the process parameters were set as follows:

| Batch No. | Zone 2 | Zone 3 | Zone 4 | Zone 5 | Zone 6 | Zone 7 | Zone 8 | Nozzle | Screw speed | Torque (N.m) |
|---|---|---|---|---|---|---|---|---|---|---|
| C6620060901 | 85°C | 120 °C | 165°C | 165°C | 165°C | 165°C | 145°C | 145 °C | 100 rpm | 2.5-3.5 |
| C6620060902 | 85 °C | 120 °C | 150°C | 150°C | 150°C | 150°C | 140 °C | 140 °C | 100 rpm | 3.0-4.0 |

Two batches of samples were discharged from the nozzle in a form of a light yellow transparent colloid, and quickly cooled and solidified into a light yellow transparent solid, which were collected in a stainless steel tray, and broken into small segments, and appropriate amounts of the samples were pulverized using a universal grinder and passed through a 100-mesh standard sieve to obtain the solid dispersion.

### Example 4 X-ray powder diffraction (XRPD) spectrum of solid dispersion of curcumin derivative C66:

### Measurement instrument: Smart Lab-3 kW Powder X-Ray Diffractometer purchased from Rigaku Corporation.

### Measurement conditions:

| | | | |
|---|---|---|---|
| Scanning mode: | Continuous scanning | Drive mode: | 0-2θ linkage |
| Starting angle: | 3° | Ending angle: | 50° |
| Scanning speed: | 0.02°/sec | Sampling time: | 1 sec |
| Target material: | Cu | Tube voltage: | 40 kV |
| Tube current: | 30 mA | | |

The sample was transferred onto a zero-background XRPD sample holder and gently pressed and ground to ensure that a surface of the sample under test was smooth and uniform.

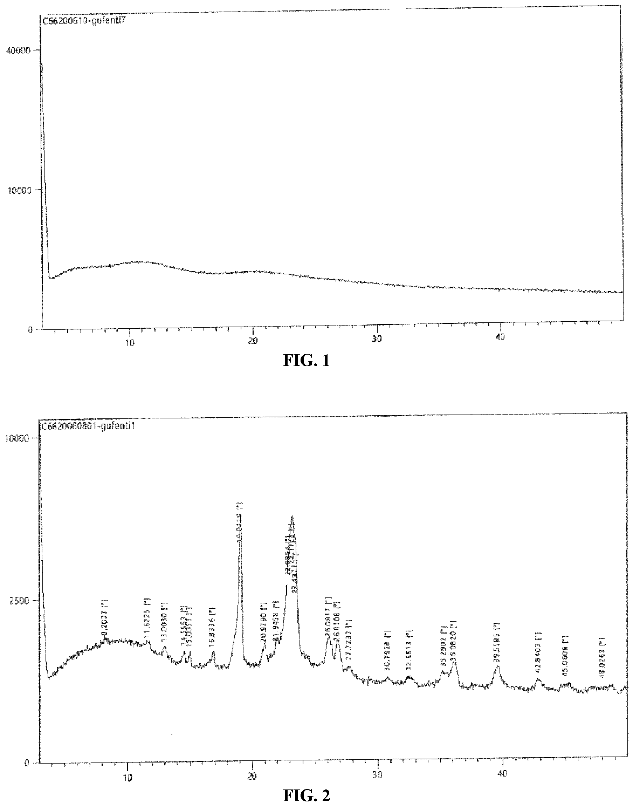

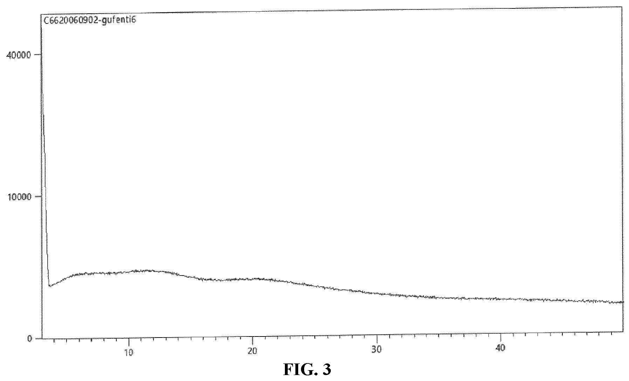

FIG. 1 is an X-ray diffraction pattern of the C66 solid dispersion prepared in Example 1, the pattern indicates that the C66-povidone K30 solid dispersion has no sharp diffraction peak in a range of 5°-30°, and diffraction signals are relatively smooth, proving that the solid dispersion is an amorphous solid dispersion. FIG. 2 is an X-ray diffraction pattern of the C66 solid dispersion prepared in Example 2. Unlike FIG. 1, the diffraction pattern of the C66-polyethylene glycol 6000 solid dispersion has many sharp diffraction peaks. Comparative analysis confirms that the diffraction peaks originate from C66 and polyethylene glycol 6000. FIG. 2 shows that the C66-polyethylene glycol 6000 solid dispersion is a microcrystalline solid dispersion, which differs from the amorphous solid dispersion of Example 1. FIG. 3 is an X-ray diffraction pattern of the C66-copovidone S630 solid dispersion prepared in Example 3 using the hot melt extrusion technology. Similar to FIG. 1, no sharp diffraction peaks are identified in FIG. 3, indicating that the solid dispersion is also an amorphous solid dispersion.

### Example 5 Actual solubilization effect of solid dispersion of curcumin derivative C66

Dissolution rate of the C66-copovidone S630 solid dispersion prepared in Example 3 in a pH 1.0 solution of 1% SDS:
Dissolution and Release Rate Determination Method II (Paddle Method), General Rule 0931 of Part IV in *Chinese Pharmacopoeia* (ChP 2020): a rotation speed of 75 rpm, a medium volume of 900 mL, and a sampling and replenishing volume of 10 mL.

Dissolution medium: a pH 1.0 solution of 1% SDS (1 g of SDS was taken, an appropriate amount of degassed purified water was added, stirred and dissolved, 9 mL of hydrochloric acid was added, and then degassed purified water was added to make up to 1000 mL, and mixed thoroughly to obtain the dissolution medium).

Time points for sampling: 15, 30, 45, and 60 min.

Detection method: 0401 Ultraviolet-visible spectrophotometry of Part IV in *Chinese Pharmacopoeia* (ChP 2020), with a detection wavelength of 305 nm.

Reference standard stock solution: 20 mg of self-prepared C66 reference standard was accurately weighed and placed into a 50 mL volumetric flask, an appropriate amount of acetonitrile was added to dissolve the reference standard, and made up to volume with acetonitrile and mixed well to obtain the stock solution.

Reference standard solution: 1 mL of the reference standard stock solution was accurately weighed and transferred into a 50 mL volumetric flask, and the dissolution medium was added to make up to a fixed volume, and mixed well to obtain the reference standard solution.

Test solution: 10 mL of the sample solution was taken manually, filtered through a 0.45 µm membrane, 3 mL of the solution was discarded, and a filtrate was collected to obtain the test solution.

### Test results:

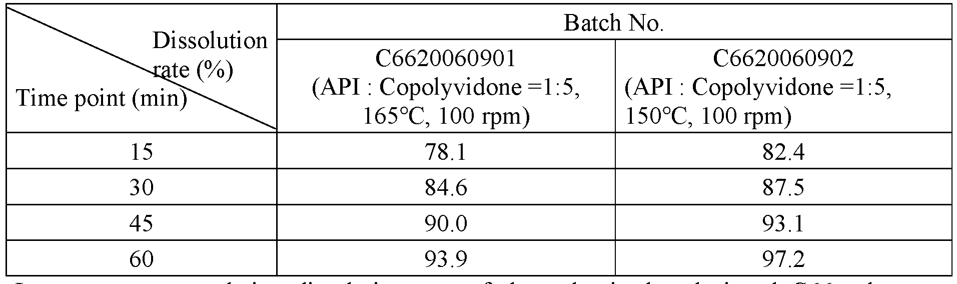

In contrast, a cumulative dissolution rate of the pulverized and sieved C66 substance (particle size D0.9 < 15 µm) in 3% sodium dodecyl sulfate (SDS, anionic surfactant) aqueous solution was 25.3% in 60 min; a cumulative dissolution rate of in different concentrations of triton X-100 (non-ionic surfactant) aqueous solution was 26.3% in 60 min; and a maximum cumulative dissolution rate in different concentrations of hexadecyl trimethyl ammonium bromide (CTAB, cationic surfactant) aqueous solution was 18.9% in 60 min. None of them reached the quality control requirement that the cumulative dissolution rate could be equal to or greater than 85% in 30 min - 60 min. Therefore, preparing C66 as a solid dispersion could significantly increase the dissolution rate of C66.

### Example 6 Effect of solid dispersion of curcumin derivative C66 on oral bioavailability of C66

### (1) Plasma detection method

Plasma samples were analyzed using an AB Sciex 5500 (LC-MS/MS) with an electrospray ionization source (ESI), and monitored with a multiple reaction monitoring (MRM) scanning method in a positive ion mode. A gradient elution was performed with 0.1% formic acid aqueous solution-acetonitrile as a mobile phase. Unknown samples were subjected to protein precipitation using methanol before injection, with a flow rate of 0.3 mL/min. Chromatographic column: Kinetex^{®} 1.7 µm, XB-C18 2.1*100 mm. Guard column: Phenomenex UHPLC C18 2.1 mm. A linear range of C66 was 1.000-288.0 ng/mL, with a lower limit of quantitation of 1.000 ng/mL.

### (2) Test method

9 male Beagle dogs (with a weight of 8.00-8.62 kg per dog) that passed the quarantine were selected and divided into 3 groups by weight according to a completely random method, with 3 dogs in each group, that is, oral administration group 1, oral administration group 2 (the solid dispersion sample obtained in Example 3), and Intravenous injection group, and single gavage administration were given. The dogs were subjected to fasting but not water deprivation 12 h before administration, and were provided with food 4 h after administration, with free access to water.

The dosage design for each group was as follows:

| Group | Test sample | Dose (mg/kg) | Concentration (mg/mL) | Volume (mL/kg) |
|---|---|---|---|---|
| Oral administration group 1 | C66 substance | 3 | 0.6 | 5 |
| Oral administration group 2 | C66 solid dispersion | 3 | 0.6 | 5 |
| Intravenous injection group | C66 substance | 1 | 1 | 1 |

| | | | | |
|---|---|---|---|---|
| Notes: The dosage of C66 solid dispersion was calculated based on the substance. | | | | |

Blood collection time points:
For the oral administration groups, the blood collection time points were before administration and 15 min, 30 min, 45 min, 1 h, 2 h, 3 h, 4 h, 6 h, 8 h, 10 h and 24 h after administration, and 0.5-1.0 mL of blood were collected from a small saphenous vein outside a hind limb or a cephalic vein inside a forelimb, and placed into a heparin sodium anticoagulation tube. For the Intravenous injection group, the blood collection time points were before administration and 5 min, 15 min, 30 min, 45 min, 1 h, 2 h, 3 h, 4 h, 8 h, 10 h and 24 h after administration, and 0.5-1.0 mL of blood were collected from a small saphenous vein outside a hind limb or a cephalic vein inside a forelimb, and placed into a heparin sodium anticoagulation tube. Main pharmacokinetic parameters such as AUC₀₋ₜ, AUC_{0-∞} and t_{1/2} were calculated using a Phoenix WinNonlin 8.0 software.

Preparation method of the test sample:
Oral administration group 1: A required amount of C66 substance was weighed, and an appropriate amount of 1% CMC-Na was added to prepare a 0.6 mg/mL suspension.

Oral administration group 2: A required amount of C66 solid dispersion was weighed, and an appropriate amount of 1% CMC-Na was added to prepare a 0.6 mg/mL suspension.

Intravenous injection group: A required amount of C66 substance was weighed and dissolved in a solution containing 5% DMSO + 10% polyethylene glycol-15 hydroxystearate + 85% (20% 2-hydroxypropyl-β-cyclodextrin) to prepare a 1 mg/mL clear solution. The solution was prepared on an exact same day of administration, and a specific preparation volume was calculated based on the body weight of the dogs when they were grouped.

### (3) Test results

After oral gavage administration of C66 substance and C66 solid dispersion at a dose of 3 mg/kg to the Beagle dogs, C66 was rapidly absorbed in vivo, and the time to reach peak concentration (Tmax) was basically the same, reaching the peak in about 1.0 h. In addition, it was also eliminated quickly in vivo, with a half-life (t_{1/2}) of approximately 1.0 h for the oral administration groups and approximately 0.5 h for the Intravenous injection group; and ratios of AUC₀₋ₜ to AUC_{0-∞} for the oral administration group 1, oral administration group 2, and the Intravenous injection group were all greater than 80% (88%, 99%, and 99%, respectively), indicating that the blood collection time points were set reasonably, effectively taking in account a absorption phase, an equilibrium phase, and an elimination phase.

After intravenous injection of C66 substance at a dose of 1 mg/kg to the Beagle dogs, Animal 3M01 vomited yellow gastric fluid 11 min after administration, laid down in the cage 19 min after injection, and recovered to normal status 53 min after injection; and Animal 3M03 laid down and experienced rapid breathing 13 min after injection, and recovered to normal status 43 min after injection.

Calculated by AUC₀₋ₜ, the oral bioavailability of C66 substance was 7.16%, and the oral bioavailability of C66 solid dispersion was 47.66%. The oral bioavailability of C66 solid dispersion was 6.66 times that of C66 substance.

Specific parameter results were shown in Tables 3 and 4.

**Table 3 Main pharmacokinetic parameters of C66 substance and solid dispersion after oral administration and intravenous injection in Beagle dogs**

| Group | Test sample | Animal No. | Main pharmacokinetic parameters | | | | | | | |
|---|---|---|---|---|---|---|---|---|---|---|
| | | | t_{1/2} (hr) | Tₘₐₓ (hr) | Cₘₐₓ (ng/mL) | AUC₀₋ₜ (hr* ng/mL) | AUC_{0-∞} (hr* ng/mL) | Vz_F_obs (mL/kg) | Cl_F_obs (mL/hr/kg) | MRTₗₐₛₜ (hr) |
| Oral administration group 1 (3 mg/kg) | C66 substance | 1M01 | 1.050 | 1 | 12.37 | 20.836 | 25.844 | / | / | 1.537 |
| | | 1M02 | 0.731 | 2 | 19.28 | 42.716 | 45.770 | / | / | 1.886 |
| | | 1M03 | 1.886 | 1 | 1.928 | 2.207 | 5.839 | / | / | 1.265 |
| Oral administration group 2 (3 mg/kg) | C66 solid dispersion | 2M01 | 1.857 | 1 | 55.02 | 164.248 | 168.085 | / | / | 2.389 |
| | | 2M02 | 0.732 | 1 | 199.0 | 268.923 | 270.556 | / | / | 1.516 |
| | | 2M03 | 0.837 | 1 | 95.41 | 201.384 | 202.621 | / | / | 1.936 |
| Intravenous injection group (1 mg/kg) | C66 substance | 3M01 | 0.562 | 0.083 | 333.9 | 161.699 | 163.139 | 4970.463 | 6129.752 | 0.684 |
| | | 3M02 | 0.476 | 0.083 | 317.6 | 137.392 | 138.027 | 4971.294 | 7244.939 | 0.536 |
| | | 3M03 | 0.483 | 0.083 | 391.9 | 144.675 | 145.268 | 4799.059 | 6883.809 | 0.574 |

| | | | | | | | | | | |
|---|---|---|---|---|---|---|---|---|---|---|
| Note: "/" means it cannot be calculated. | | | | | | | | | | |

**Table 4 Main pharmacokinetic parameters of C66 substance and solid dispersion after oral administration and intravenous injection in Beagle dogs**

| | | C66 | | | | | |
|---|---|---|---|---|---|---|---|
| | | Oral administration group 1 (3 mg/kg) | | Oral administration group 2 (3 mg/kg) | | Intravenous injection group (1mg/kg) | |
| Variab le | Unit | Mean * | SD* | Mean | SD | Mean | SD |
| AUC_{0-∞} | hr*ng/m L | 35.807 | 14.090 | 213.75 4 | 90.300 | 148.812 | 12.925 |
| AUC₀₋ₜ | hr*ng/m L | 31.776 | 15.471 | 211.51 8 | 91.917 | 147.922 | 12.475 |
| Cₘₐₓ | ng/mL | 15.825 | 4.886 | 116.5 | 128.6 | 347.8 | 39.1 |
| t_{1/2} | hr | 0.891 | 0.226 | 1.142 | 1.076 | 0.507 | 0.048 |
| MRTₗₐₛₜ | hr | 1.712 | 0.247 | 1.947 | 0.437 | 0.598 | 0.077 |
| Tₘₐₓ | hr | 1.500 | 0.707 | 1.000 | 0.000 | 0.083 | 0.000 |
| Absolute bioavailability (%), (AUC₀₋ₜ): | | 7.16 | | 47.66 | | NA | NA |
| Relative bioavailability (%) (AUC₀₋ₜ): | | 15.02 | | NA | | NA | NA |

| | | | | | | | |
|---|---|---|---|---|---|---|---|
| Note: The calculation formula of absolute bioavailability is: F (AUC₀₋ₜ) = AUC₀₋ₜ oral * Dose intravenous/AUC₀₋ₜ intravenous * Dose oral × 100%; the calculation formula of relative bioavailability is: F (AUC₀₋ₜ) = AUC₀₋ₜ oral administration group 1 * Dose oral administration group 2/AUC₀₋ₜ oral administration group 2 * Dose oral administration group 1 × 100%; "*" indicates that the results of Animal 1M03 are very different from those of the other two animals, and the Mean and SD calculation of this group are not included. | | | | | | | |

### Example 7 Anti-aging and stability of solid dispersion of curcumin derivative C66

Storage conditions at room temperature: plastic sampling bag packaging, sealed, room temperature, light-proof storage;

Acceleration test conditions: aluminum foil bag packaging, sealed, 40 ± 2°C, 75% ± 5% RH, sample placement;

Samples: Solid dispersions obtained in Examples 1, 2, and 3.

### Dissolution curve test results (test conditions are the same as above):

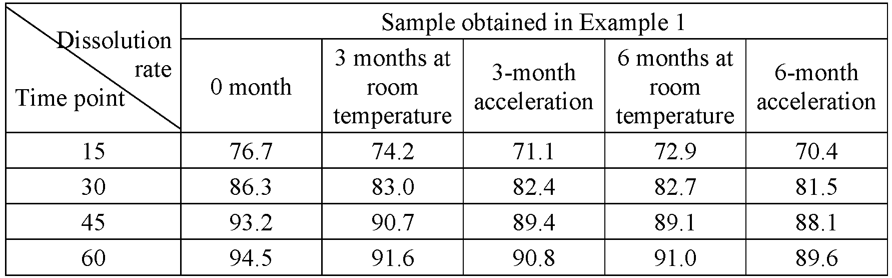

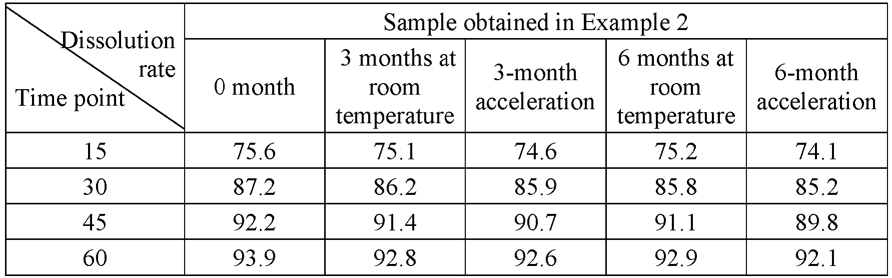

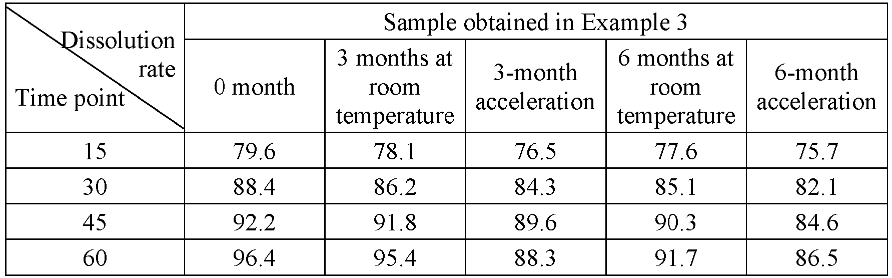

### Determination results of related substances:

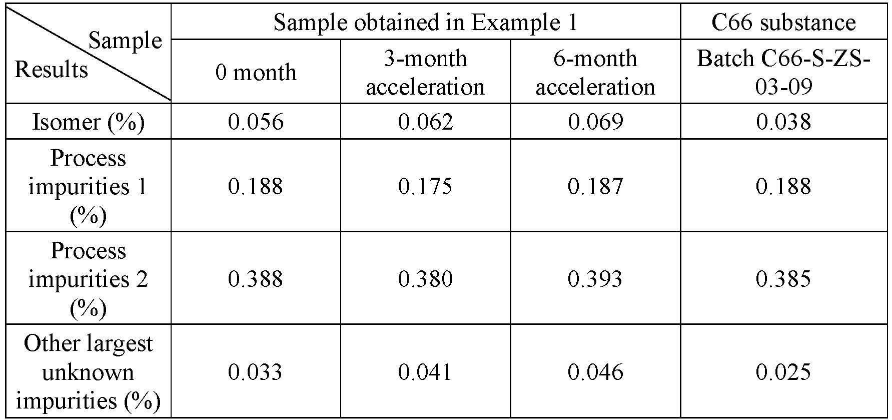

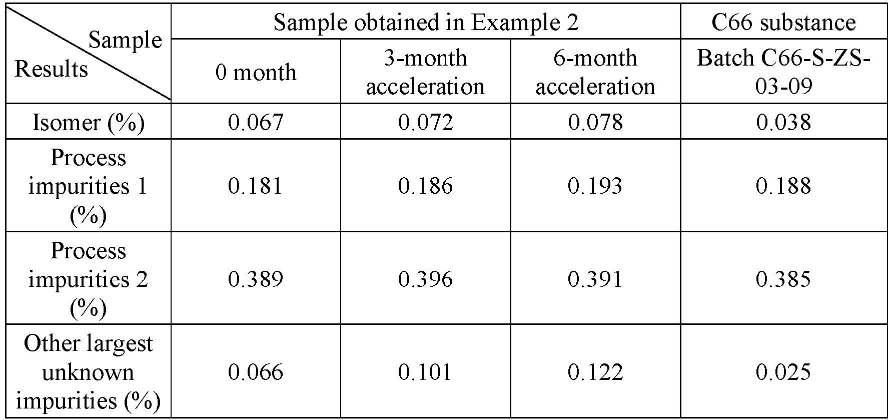

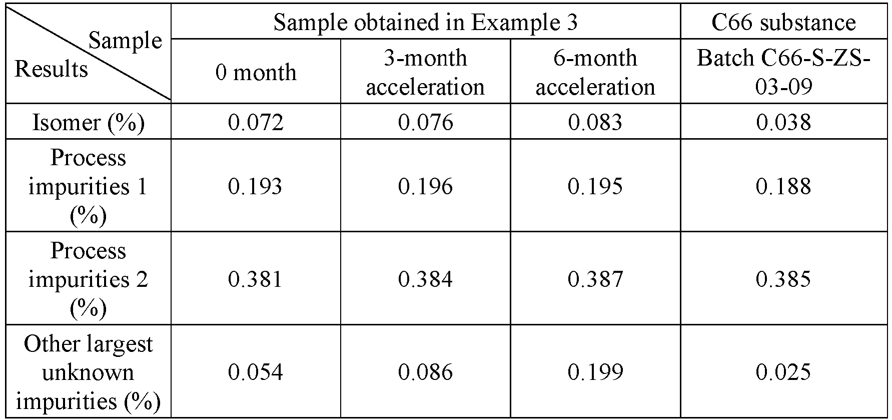

### Content determination results:

| Sample | Sample obtained in Example 1 | | | C66 substance |
|---|---|---|---|---|
| | 0 month | 3-month acceleration | 6-month acceleration | Batch C66-S-ZS-03-09 |
| Content (%) | 97.6 | 97.3 | 97.2 | 98.5 |

| Sample | Sample obtained in Example 2 | | | C66 substance |
|---|---|---|---|---|
| | 0 month | 3-month acceleration | 6-month acceleration | Batch C66-S-ZS-03-09 |
| Content (%) | 98.2 | 98.0 | 98.1 | 98.5 |

| Sample | Sample obtained in Example 3 | | | C66 substance |
|---|---|---|---|---|
| | 0 month | 3-month acceleration | 6-month acceleration | Batch C66-S-ZS-03-09 |
| Content (%) | 97.9 | 97.6 | 97.5 | 98.5 |

### Results analysis:

After 6 months of storage under both room temperature and acceleration conditions, the C66 solid dispersions obtained in Examples 1, 2, and 3 showed no significant changes in content or related substances, the dissolution rates slightly slowed down, but the cumulative dissolution rates exceeded 80% in 45 min, and exceeded 85% in 60 min, indicating that the dispersions remained basically stable, and no obvious aging was commonly observed in other solid dispersions. More specifically, after 6 months of storage under both room temperature and acceleration conditions, the solid dispersion obtained in Example 2 showed the lowest decrease in the dissolution rate, followed by the solid dispersion in Example 1, and the solid dispersion obtained in Example 3 showed the largest decrease in the dissolution rate, indicating that although the dissolution rates of the solid dispersions obtained in Examples 1, 2, and 3 still met the quality requirements after 6 months of acceleration test, but they showed slight differences in the anti-aging performance.

In summary, the C66 solid dispersions obtained in Examples 1, 2, and 3 met the quality control requirements in terms of various key quality attributes after room temperature and acceleration stability tests.

### Example 8 Determination of angle of repose

### Method: Funnel method

Process: A glass culture dish was taken, and a radius (R) of the culture dish was measured; a glass funnel was taken and fixed on a wooden stand, the culture dish was placed upside down below the funnel, a funnel outlet was aligned with a center of a culture dish bottom plate, and a distance between the funnel outlet and the center of the culture dish bottom plate was 4.0-5.0 cm. An appropriate amount of the C66 solid dispersion material obtained in Example 3 was slowly added from a top of the funnel, and gradually accumulated on the culture dish bottom plate through buffer of the funnel to form a cone until a highest cone was obtained, and a height (H) of the cone was measured.

Calculation formula: Angle of repose α= arctan (H/R). Measurement was repeated 3 times and an average value was taken. Results were shown in Table 6.

**Table 6 Determination of angle of repose**

| 1 | 2 | 3 | Mean |
|---|---|---|---|
| 41.80° | 42.20° | 41.93° | 41.98° |

The average angle of repose was 41.98°, indicating that the C66 solid dispersion has good fluidity and can be conveniently used in various formulation processes.

### Example 9 Scale-up preparation of solid dispersion of curcumin derivative C66

Based on the small-scale test formulation process obtained in the previous test, C66 substance and polyethylene glycol 6000 were weighed and premixed evenly according to a feed ratio of C66 substance: polyethylene glycol 6000 = 1: 12 (g/g). A hot-melt extruder was used to prepare the solid dispersion, pulverized and sieved to obtain a C66 solid dispersion. 2.3 kg of C66 CPI and 27.6 kg of polyethylene glycol 6000 were used. Main equipment used included a hot-melt extruder (Pharma 24, Thermo), a hammer mill (D6A, Fitzpatrick), and a universal grinder (40B, Jiangsu Guibao Group). This example was carried out in a GMP production facility.

After the hot-melt extruder accessories were installed and debugged, and turned on to heat to a preset value, a screw was started, and materials were added to prepare the solid dispersion. The materials were collected in stainless steel trays, about 0.2 kg per tray, the materials were cooled and solidified; and a stainless steel spatula was used to scoop it out and set aside for further use. Temperature parameters of the hot-melt extruder were set as follows:

| Zone 2 | Zone 3 | Zone 4 | Zone 5 | Zone 6 | Zone 7 | Zone 8 | Nozzle | Feed speed | Screw speed |
|---|---|---|---|---|---|---|---|---|---|
| 40°C ± 5°C | 85°C ± 5°C | 115°C ± 5°C | 128°C ± 5°C | 128°C ± 5°C | 128°C ± 5°C | 85°C ± 5°C | 65°C ± 5°C | 50 Hz | 50 - 100 rpm |

### Pulverizing:

Pulverizing 1: the solidified sheets were placed in the hammer mill and pulverized with a back of the blade, with a sieve with a pore size of 0.079 in and a rotor speed of 5000 rpm.

pulverizing 2: A 40B universal grinder (80-mesh sieve) was turned on at a pulverizing speed of 50 Hz, and the materials after pulverizing were then sieved through an 80-mesh screen to obtain a final product.

The materials after pulverizing and sieving were light yellow powder, which were consistent with the small-scale test samples. The sample detection content was 101.6% ±1.1% of a theoretical value. Results of related substance detection showed that an isomer content of the C66 solid dispersion scaled-up production sample was slightly higher than that of the substance, but both were lower than 0.2%. However, there were no significant differences in process-related impurities, indicating that the related substances of the scale-up sample met the requirements. In addition, a dissolution rate of the scaled-up sample exceeded 90% in 60 min, which was the small-scale sample, meeting a target dissolution rate of more than 85% in 60 min. This example indicated that the process of scale-up production of C66 solid dispersion was successful, main detection indicators showed good reproducibility with the small-scale sample, demonstrating the feasibility of the scale-up production.

### Example 10 Significant reduction in dissolution rate of solid dispersion of curcumin derivative C66 with PEG 4000 and Poloxamer 188 as carrier after storage

(1) 2.05 g of C66 substance and 10.08 g of hydroxypropyl methylcellulose acetate succinate (HPMCAS) were weighed and placed into a stoppered conical flask, 50 mL of acetone and 50 mL of ethanol were respectively taken and added to the stoppered conical flask to obtain a mixture, the flask was sealed tightly, the mixture was dissolved by ultrasonic shaking until clear to obtain a mixed solution 1 for later use;
(2) 2.01 g of C66 substance and 10.10 g of PEG 4000 were weighed and placed into a stoppered conical flask, 50 mL of acetone and 50 mL of ethanol were respectively taken and added to the stoppered conical flask to obtain a mixture, the flask was sealed tightly, the mixture was dissolved by ultrasonic shaking until clear to obtain a mixed solution 2 for later use;
(3) 2.03 g of C66 substance and 10.13 g of Poloxamer 188 were weighed and placed into a stoppered conical flask, 50 mL of acetone and 50 mL of ethanol were respectively taken and added to the stoppered conical flask to obtain a mixture, the flask was sealed tightly, the mixture was dissolved by ultrasonic shaking until clear to obtain a mixed solution 3 for later use;
(4) a Buchi B290 spray dryer, with a nozzle size of 1.5 mm, was used for drying, an inlet air temperature was 60-65°C, a peristaltic pump feed rate was set to 15 mL/min, and an outlet air temperature was 35-40°C; and the outlet air temperature was used as a main control parameter, and was maintained with the ranged by adjusting the inlet air temperature; and
(5) sample powder in a cyclone collector was scraped and taken, and the powder was powder was the C66 solid dispersions prepared with different carriers, and sealed for storage. The C66 solid dispersions obtained in the steps (1), (2) and (3) were designated as Solid Dispersion 1, 2, and 3, respectively;
(6) Solid Dispersions 1, 2, and 3 were subjected to solubility tests according to the dissolution curve test method described in Example 5; and
(7) Solid Dispersions 1, 2 and 3 were placed under the acceleration test conditions described in Example 7 (aluminum foil bag packaging, sealed, 40±2°C, 75%±5%RH) for 3 months, and dissolution of the dispersions was detected and compared with the dissolution results before sampling, to analyze whether the sample had a phenomenon of decreased dissolution rate. The test results were itemized in the table below:

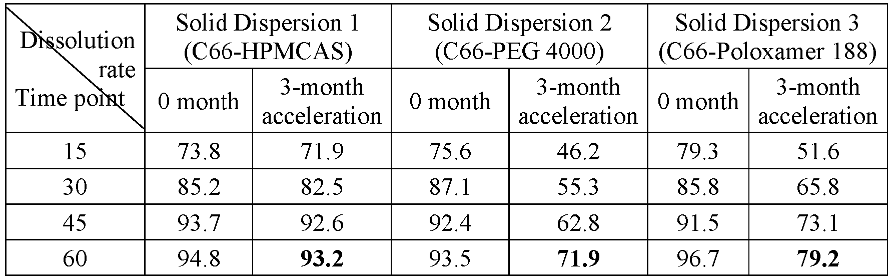

From the results, it can be seen that a C66 dissolution rate of Solid Dispersion 1 with the HPMCAS as the carrier did not show a significant decrease after being stored under acceleration test conditions for 3 months. In contrast, Solid Dispersions 2 and 3 using PEG 4000 and Poloxamer 188 as carriers dissolved faster at 0 month, meeting the quality control requirements. However, after storage for 3 months, the dissolution rates decreased significantly, with cumulative dissolution rates in 60 min being only 71.9% and 79.2%, respectively, which no longer met the quality control requirements of cumulative dissolution more than 85% in 30 min - 60 min. Therefore, although the solid dispersion technology could increase the dissolution rate of C66, the appropriate selection of the carrier played a critical role in the preparation of C66 solid dispersions with anti-aging capability.

### Example 11: Effect of different mass ratios of C66 to PEG 6000 on the anti-aging capability of C66 solid dispersions

### Prescription composition:

| Mass ratio of C66 to PEG 6000 | Prescribed amount (g) | Remarks |
|---|---|---|
| 1:5 | 25 g : 125 g | C66 and PEG 6000 pass through a 65-mesh standard sieve after universal grinding |
| 1:12 | 11.5 g : 138.5 g | |
| 1:20 | 7.1 g : 142.9 g | |

C66-PEG 6000 solid dispersions were prepared according to the process described in Example 2, and the samples were prepared with mass ratios of C66 to PEG 6000 being 1:5, 1:12, and 1:20 and designated as Solid Dispersions 1, 2, and 3, respectively. Solid Dispersions 1, 2, and 3 were subjected to solubility tests according to the dissolution curve test method described in Example 5; and Solid Dispersions 1, 2 and 3 were placed under the acceleration test conditions described in Example 7 (aluminum foil bag packaging, sealed, 40±2°C, 75%±5%RH) for 3 months, and dissolution of the dispersions was detected and compared with the dissolution results before sampling, to analyze the aging of the samples after long-term storage. The experimental results were itemized in the table below:

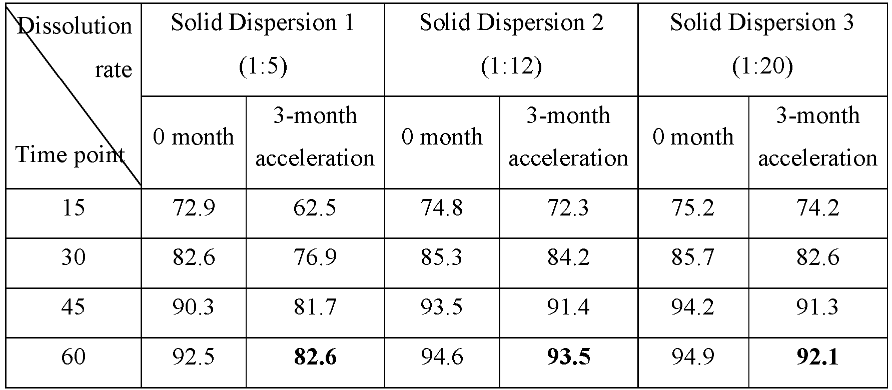

From the results, it can be seen that the dissolution rates of the three C66 solid dispersions with different mass ratios all met the expected requirements immediately after preparation (0 month), with dissolution rates exceeding 90% in 60 min. However, after storage under acceleration conditions for 3 months, the dissolution rates changed, specifically, cumulative dissolution rates of Solid Dispersions 1, 2, and 3 in 60 min decreased by 9.9%, 1.1%, and 2.8%, respectively, compared with those at 0 month. The decline in dissolution rate of Solid Dispersion 1 was significantly greater than that of Solid Dispersions 2 and 3, with the dissolution rate falling below 85% in 60 min, which no longer met the quality requirements. In contrast, the dissolution rates of Solid Dispersions 2 and 3 were essentially unaffected after storage for 3 months under acceleration conditions. Therefore, the aging speed of solid dispersions prepared using the same carrier and C66 at different mass ratios was different, indicating that the amount and proportion of the carrier played an important role in the anti-aging performance of C66 solid dispersions. In addition, although Solid Dispersion 3 exhibited good anti-aging performance, its drug loading (4.8%) was significantly lower than that of Solid Dispersion 2 (7.7%), which could lead to the problem of excessive final volume of clinical medication. Considering both the anti-aging performance and drug loading, Solid Dispersion 2 demonstrated the best drug performance in this example.

### Example 12 Preparation of solid dispersion capsules of curcumin derivative C66

Lactose monohydrate was passed through a 200-mesh sieve, and the C66 solid dispersion prepared in Example 9 was passed through a 100-mesh sieve for later use. 416.0 g of 200-mesh lactose monohydrate, 440.0 g of microcrystalline cellulose (SH101), 4.0 g of magnesium stearate, and a solid dispersion containing 40.0 g of C66 were accurately weighed. All the raw and auxiliary materials were placed into a square cone mixer and mixed evenly for 5 min to obtain a mixture, the mixture was then taken out and added to a hopper of a fully automatic capsule filling machine, 2^{#} gelatin hollow capsule shells were used for filling, with a theoretical fill weight of 225 mg per capsule. The filling process was smooth, a weight difference of the capsules was lower than 3%, and a content of the main drug C66 content was ±3% of a predetermined dosage specification (10 mg), such that a filling accuracy met the quality requirements.

### Example 13 Preparation of solid dispersion tablets of curcumin derivative C66

Lactose monohydrate was passed through a 200-mesh sieve, and the C66 solid dispersion prepared in Example 3 was passed through a 100-mesh sieve for later use. 176.0 g of sieved lactose monohydrate, 870.0 g of microcrystalline cellulose (SH101), 5.0 g of magnesium stearate, 22.6 g of cross-linked sodium carboxymethyl cellulose, and a solid dispersion containing 60.0 g of C66 were accurately weighed. All the raw and auxiliary materials were placed into a square cone mixer and mixed evenly for 5 min to obtain a mixture, the mixture was taken out and added to a hopper of a fully automatic tablet press and pressed into tablets using a 9 mm round convex punch, with a theoretical tablet weight of 189 mg. The tableting process was smooth, a stable production pressure was relatively stable, and the tablet weight was controlled within ±3%. A hardness of the tablets was 6-9 kg, and a content of C66 content was controlled within ±3%. The C66 solid dispersion met the requirements of direct powder compression process and produced tablets with acceptable quality.

## Claims

1. A solid dispersion of a curcumin derivative, **characterized by** comprising a curcumin derivative and a carrier material;
the curcumin derivative is C66, with a structure shown in Formula (I): 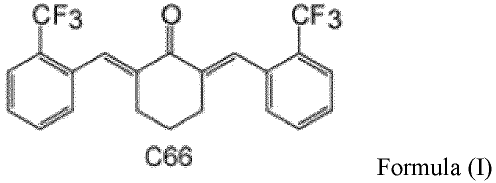
the carrier material comprises one, two, or three of polyethylene glycol 6000, povidone K30, copovidone S630, hydroxypropyl methylcellulose acetate succinate, hydroxypropyl methylcellulose; and
the curcumin derivative is dispersed in the carrier material in a microcrystalline or amorphous form.

2. The solid dispersion of the curcumin derivative according to claim 1, **characterized in that** a mass ratio of the curcumin derivative to the carrier material is 1:0.5 to 1:25.

3. A method for preparing the solid dispersion of the curcumin derivative according to claim 1 or 2, **characterized in that** the curcumin derivative is dispersed in the carrier material to obtain the solid dispersion; and
a method for dispersion is a spray drying method, a hot-melt extrusion method, or a melt method for dispersion.

4. The method for preparing the solid dispersion of the curcumin derivative according to claim 3, **characterized in that** the method for dispersion is the spray drying method, with a specific process as follows:
dissolving or dispersing the curcumin derivative and the carrier material in a solvent to obtain a curcumin derivative-carrier material solvent dispersion system, and then drying the curcumin derivative-carrier material solvent dispersion system using a spray dryer to obtain the solid dispersion of the curcumin derivative; **characterized in that**
the solvent comprises one or a mixed solvent of two or more of water, ethanol, and acetone.

5. The method for preparing the solid dispersion of the curcumin derivative according to claim 4, **characterized in that** the carrier material is povidone K30, and a mass ratio of the curcumin derivative to the carrier material is 1:0.5 to 1:25;
the solvent is a mixed solvent of ethanol and acetone, and a volume ratio of the ethanol to the acetone is 1:0.5 to 1.5; and
an inlet air temperature for the spray drying is 60-70°C, and an outlet air temperature for the spray drying is 30-40°C.

6. The method for preparing the solid dispersion of the curcumin derivative according to claim 3, **characterized in that** the method for dispersion is the hot-melt extrusion method, with a specific process as follows:
mixing the curcumin derivative and the carrier material to obtain a mixture, placing the mixture in a hot-melt extruder for hot-melt extrusion, and cooling, pulverizing and sieving to obtain the solid dispersion of the curcumin derivative; and
the cooling is cooling at room temperature or under low-temperature conditions; and a method for the pulverizing is shearing or grinding.

7. The method for preparing the solid dispersion of the curcumin derivative according to claim 6, **characterized in that** the carrier material is copovidone S630;
a mass ratio of the curcumin derivative to the carrier material is 1:4 to 7.
a temperature of each zone in the hot-melt extruder is 80-165°C; and
a nozzle temperature is 130-150°C.

8. A C66 solid dispersion capsule, **characterized in that** the capsule is prepared from the solid dispersion in any one of claims 1-7, together with a filler and a lubricant; and an amount of the filler accounts for 20-90% of a total weight of the capsule, and an amount of the lubricant accounts for 0.1-5% of the total weight of the capsule;
the filler comprises one or more of microcrystalline cellulose, lactose, and pregelatinized starch; and
the lubricant comprises one or more of magnesium stearate, talcum powder, and microsilica gel.

9. A method for preparing the C66 solid dispersion capsule in claim 8, **characterized by** comprising: taking the solid dispersion, mixing the solid dispersion, the filler and the lubricant uniformly, and filling into a capsule to obtain the C66 solid dispersion capsule.

10. A C66 solid dispersion tablet, **characterized in that** the tablet is prepared from the solid dispersion in any one of claims 1-7, together with a filler, a disintegrant and a lubricant; and an amount of the filler accounts for 20-90% of a total weight of the tablet, an amount of the disintegrant accounts for 0.5-15% of the total weight of the
tablet, and an amount of the lubricant accounts for 0.1-5% of the total weight of the tablet;
the filler comprises one or more of microcrystalline cellulose, lactose, and pregelatinized starch; and
the disintegrant comprises one or more of cross-linked sodium carboxymethyl cellulose, low-substituted hydroxypropyl cellulose, sodium carboxyl methylstarch, and crospovidone; and
the lubricant comprises one or more of magnesium stearate, talcum powder, and microsilica gel.

11. A method for preparing the C66 solid dispersion tablet in claim 10, **characterized by** comprising: taking the solid dispersion, mixing the solid dispersion, the filler, the disintegrant and the lubricant uniformly, and pressing into a tablet to obtain the C66 solid dispersion tablet.

12. Use of the solid dispersion, the solid dispersion capsule, or the solid dispersion tablet in any one of claims 1-11 in the preparation of a drug for treating or preventing kidney diseases or heart diseases.

13. The use according to claim 12, **characterized in that** the kidney diseases are diabetic nephropathy, hypertensive nephropathy, nephrotic syndrome, chronic glomerulonephritis, IgA nephropathy, lupus nephritis, hepatitis B-related nephritis, Henoch-Schonlein purpura nephritis, membranous nephropathy, and various primary and secondary chronic kidney diseases after kidney transplantation.
